# EUROPEAN PATENT SPECIFICATION

(11) **EP 3 666 239 B1**
(45) Date of publication and mention of the grant of the patent: **26.04.2023**
(21) Application number: 18843667.9
(22) Date of filing: 19.06.2018
(51) Int. Cl.: A61F 13/551, A61F 13/58

(54) **UNDERPANTS-TYPE DISPOSABLE DIAPER**
HOSENARTIGE WEGWERFWINDEL
COUCHE JETABLE DE TYPE CULOTTE

(30) Priority: 08.08.2017 JP 2017153728; 18.05.2018 JP 2018096511
(43) Date of publication of application: 17.06.2020
(73) Proprietor: Kao Corporation, Chuo-Ku Tokyo 103-8210 (JP)
(72) Inventor: TAKAHASHI, Akio, Haga-gun Tochigi 321-3497 (JP); OKUDA, Yasuyuki, Haga-gun Tochigi 321-3497 (JP)
(74) Representative: Vossius & Partner Patentanwälte Rechtsanwälte mbB
(86) International application number: PCT/JP2018/023216
(87) International publication number: WO 2019/031069

(56) References cited:
- EP-A1- 2 805 700
- WO-A1-01/13842
- WO-A1-2019/208178
- WO-A1-2019/208179
- JP-A- H07 157 728
- JP-A- H09 194 801
- JP-A- H09 316 406
- JP-A- H11 332 910
- JP-A- 2000 093 460
- JP-A- 2000 093 460
- JP-A- 2004 298 459
- JP-U- H0 677 722
- US-A- 5 234 517

## Description

### Technical Field

The present invention relates to a pull-on disposable diaper including a tape for disposal (hereinafter also referred to as "disposal tape").

### Background Art

For easy and hygienic disposal of pull-on disposable diapers, a technique of providing a disposal tape folded in three on the rear portion of a diaper has been known. However, even when a disposal tape simply folded in three is spread, it has a length only about three times the length of the folded tape, and thus the length may be insufficient depending on a position at which the disposal tape is fixed. To solve such a disadvantage, JP 9-99010 A discloses a disposal tape that is provided on the rear portion of a pull-on disposable diaper and is at least partly extensible in the longitudinal direction. JP2000093460 discloses a pull-on disposable diaper comprising: a waist opening, a pair of leg openings, a rear portion, a front portion and a disposal tab folded in Z-form.

### Summary of Invention

The present invention is a pull-on disposable diaper as defined in independent claim 1. Preferred aspects are defined in the dependent claims.

### Brief Description of Drawings

[Fig. 1] Fig. 1 is a perspective view showing an embodiment of a pull-on disposable diaper of the present invention.
[Fig. 2] Fig. 2 is a sectional view showing the structure of a disposal tape provided on the pull-on disposable diaper shown in Fig. 1.
[Fig. 3] Fig. 3 is a perspective view showing when the pull-on disposable diaper shown in Fig. 1 is made in a disposal shape.
[Fig. 4] Fig. 4 is a perspective view showing the disposal shape of the pull-on disposable diaper shown in Fig. 1.

### Description of Embodiments

According to the technique disclosed in JP 9-99010 A, the length of a disposal tape increases due to the extensibility thereof when a used diaper is rolled and disposed, and thus the disposal tape helps the diaper to maintain the rolled shape. In this case, in order to certainly maintain the rolled shape of a diaper, there is a demand for a disposal tape capable of extending as long as possible. When excessively extended, a disposal tape may exceed the maximum breaking elongation, and the extended part may break, unfortunately.

The present invention relates to an improvement of a diaper including a disposal tape and more specifically relates to a pull-on disposable diaper including a disposal tape having higher extensibility.

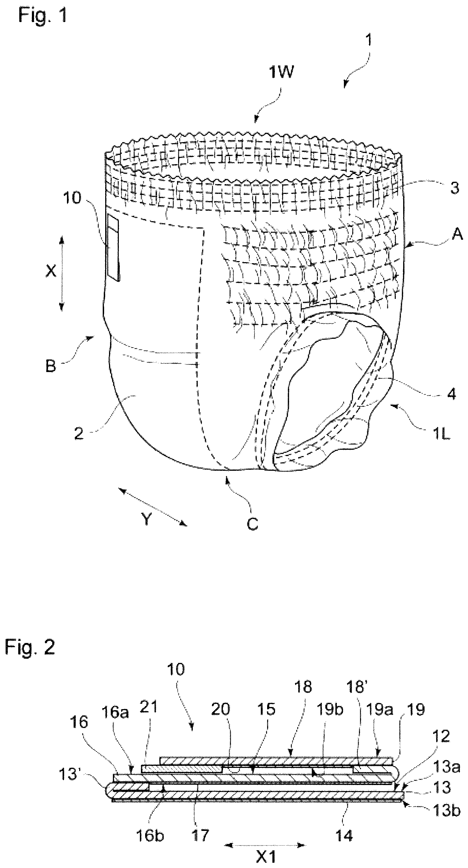

The present invention will now be described on the basis of preferred embodiments thereof with reference to drawings. Fig. 1 shows an embodiment of a pull-on disposable diaper of the present invention. The pull-on disposable diaper 1 (hereinafter also simply called "diaper 1") shown in the figure is a garment to be put on the below waist of a wearer and thus has a waist opening 1W and a pair of leg openings 1L, 1L. The diaper 1 is roughly classified into a front portion A to be placed on the front of a wearer wearing the diaper, a rear portion B to be placed on the rear of the wearer, and a crotch portion C to be placed on the crotch.

The diaper 1 has a longitudinal direction X and a width direction Y orthogonal thereto. The longitudinal direction X is coincident with the direction in which the diaper 1 is put on/off. The diaper 1 includes a topsheet (not shown) at a side to face the skin of a wearer wearing the diaper and includes a backsheet (not shown) at a side distant from the skin of the wearer. Between the topsheet and the backsheet, an absorbent member 2 is provided. The topsheet, the backsheet, and the absorbent member 2 are located at least in the whole region of the crotch portion C and extend from the crotch portion C along the longitudinal direction X to at least a part of the front portion A and to at least a part of the rear portion B.

As the topsheet, the backsheet, and the absorbent member 2, substantially the same materials as those conventionally used in the field can be used without any limitation. The diaper 1 may also include, in addition to these members, other members conventionally used in the field. For example, the diaper 1 may include, at each side of the absorbent member 2, a leak-proof cuff (not shown) extending along the longitudinal direction of the absorbent member 2.

On the rear portion B of the diaper 1, a disposal tape 10 is provided. The disposal tape 10 is used to maintain a disposal shape of the diaper 1. The disposal tape 10 is located at substantially the center in the width direction Y of the rear portion B of the diaper 1. The disposal tape 10 is provided so that the longitudinal direction thereof is coincident with the longitudinal direction X of the diaper 1.

Fig. 2 shows the cross-sectional structure of the disposal tape 10. The disposal tape 10 mainly includes three parts. Specifically, the disposal tape 10 includes a fixing part 12, an extensible part 15, and an attachment part 18. These three parts are arranged in this order along the longitudinal direction X1 (see Fig. 2) of the disposal tape 10. These three parts are folded in three stacked in this order. No member is interposed between the fixing part 12 and the extensible part 15, and these parts are directly and continuously provided. No member is also interposed between the extensible part 15 and the attachment part 18, and these parts are directly and continuously provided.

In the disposal tape 10, the fixing part 12 includes a sheet piece for a fixing part 13. The sheet piece for a fixing part 13 has a first face 13a and a second face 13b. The fixing part 12 also includes an adhesive part for a fixing part 14. The adhesive part for a fixing part 14 is provided on the second face 13b of the sheet piece for a fixing part 13. The fixing part 12 is fixed through the adhesive part for a fixing part 14 to the outer surface of the rear portion B in the diaper 1. Accordingly, the disposal tape 10 is unreleasably fixed to the outer surface of the rear portion B in the diaper 1.

In the disposal tape 10, the extensible part 15 is easily extended by a comparatively small tensile force when the tensile force is applied to the disposal tape 10, and increases the length of the disposal tape 10. The extensible part 15 includes a sheet piece for an extensible part 16. The sheet piece for an extensible part 16 may have the same length and/or the same width as those of the above-described sheet piece for a fixing part 13 or may have a different length and/or a different width from those. The sheet piece for an extensible part 16 has a first face 16a and a second face 16b. The extensible part 15 also includes an adhesive part for an extensible part 17. The adhesive part for an extensible part 17 is provided on the second face 16b of the sheet piece for an extensible part 16. The adhesive part for an extensible part 17 releasably joins the extensible part 15 to the fixing part 12 in the disposal tape 10 folded in three. Hence, the adhesive part for an extensible part 17 preferably includes a pressure-sensitive adhesive having low tackiness.

The sheet piece for a fixing part 13 in the fixing part 12 is folded at one end in the longitudinal direction X1 to the side of the extensible part 15, forming a folded part 13'. The folded part 13' is joined to one end of the sheet piece for an extensible part 16 in the extensible part 15 on the second face 16b. Accordingly, the fixing part 12 and the extensible part 15 are directly and continuously provided.

In the disposal tape 10, the attachment part 18 can be attached to any position on the outer surface of the diaper 1 and is for attaching a leading end region of the disposal tape 10 to the outer surface of the diaper 1 when a disposal shape of the diaper 1 is maintained by the disposal tape 10. The attachment part 18 includes a sheet piece for an attachment part 19. The sheet piece for an attachment part 19 may have the same length and/or the same width as those of the sheet piece for a fixing part 13 and/or the sheet piece for an extensible part 16 or may have a different length and/or a different width from those. The sheet piece for an attachment part 19 has a first face 19a and a second face 19b. The attachment part 18 also includes an adhesive part for an attachment part 20. The adhesive part for an attachment part 20 is provided on the second face 19b of the sheet piece for an attachment part 19. The adhesive part for an attachment part 20 releasably joins the attachment part 18 to the extensible part 15 in the disposal tape 10 folded in three and is to certainly attach the attachment part 18 to any position on the outer surface of the diaper 1. In consideration of this, the tackiness degree of the adhesive part for an attachment part 20 is preferably set.

The sheet piece for an extensible part 16 in the extensible part 15 is folded at one end in the longitudinal direction X1 to the side of the attachment part 18, forming a folded part 18'. The folded part 18' is joined to one end of the sheet piece for an attachment part 19 in the attachment part 18 on the second face 19b. Accordingly, the extensible part 15 and the attachment part 18 are directly and continuously provided. To the other end of the sheet piece for an attachment part 19 in the attachment part 18, a tab 21 is joined on the second face 19b.

The disposal tape 10 including the above three parts is folded in three to form a Z shape. The disposal tape 10 is so attached onto the rear portion B of the diaper 1 that a free end of the disposal tape 10 folded in three, or an attachment site of the tab 21 in the attachment part 18, points in the direction of the waist opening 1W in the diaper 1.

In the disposal tape 10, the attachment part 18 starts to extend at a tensile strength higher than the tensile strength at which the extensible part 15 starts to extend when the disposal tape 10 is pulled in the longitudinal direction X1. Accordingly, when the tab 21 in the disposal tape 10 is held with fingers, and the disposal tape 10 is pulled, the extensible part 15 extends before the attachment part 18 extends. In particular, when the tensile strength at which the extensible part 15 starts to extend is 6.5 N or less, the extensible part 15 is likely to extend prior to the extension of the attachment part 18. From this viewpoint, the tensile strength at which the extensible part 15 starts to extend is preferably 5.0 N or less. From the viewpoint of preventing unintentional extension when extension is not needed, the tensile strength is preferably 1.0 N or more. The tensile strength is determined independent of the width of a disposal tape 10. The tensile strength at which each part starts to extend is the first maximum strength determined as follows: each part is attached in the longitudinal direction to chucks of a tensile tester; the chuck distance is set at 10 mm; and the part is pulled at a rate of 300 mm/min (when the first maximum point is not clearly identified or observed, the maximum load during extension from an elongation of 10% to 50% is considered as the first maximum strength).

From the same viewpoint, in the disposal tape 10, the fixing part 12 starts to extend at a tensile strength higher than the tensile strength at which the extensible part 15 starts to extend when the disposal tape 10 is pulled in the longitudinal direction X1. The fixing part 12 is unreleasably fixed to the outer surface of the diaper 1 through the adhesive part for a fixing part 14, and thus the fixing part 12 naturally starts to extend at a tensile strength higher than the tensile strength at which the extensible part 15 starts to extend.

The extensible part 15 has such a tensile strength as described above. As for the length extended when the extensible part 15 is pulled, the extensible part 15 preferably has such an extensibility that the extended length without breakage is 150 mm or more in term of capable of certainly maintaining a disposal shape of the diaper 1 with the extended disposal tape 10. From this viewpoint, the extensible part 15 more preferably has such an extensibility that the extended length without breakage is 200 mm or more. From the same viewpoint, the extended length without breakage is preferably 300 mm or less. The extended length without breakage is the length at breakage when the respective ends of an extensible part 15 in the longitudinal direction are attached to chucks of a tensile tester, and the extensible part is pulled at a rate of 300 mm/min. The extended length includes the length before extension (the attachment part and the fixing part are attached to the chucks for the measurement).

The extensible part 15 preferably has such an extensibility that the ratio of the length after extension L2 to the length before extension L1, L2/L1, is 4 or more. This is because when an extensible part 15 is short but has such an extensibility, the extensible part 15 sufficiently extends, and thus the disposal tape 10 can have a small length. From this viewpoint, the value L2/L1 is more preferably 5 or more and more preferably 6 or more. The value is preferably 8 or less from the viewpoint of preventing excess extension. L2 has the same meaning as the above extended length without breakage.

In order to allow the extensible part 15 to have the above tensile strength or the extension, an appropriate material can be selected as the sheet piece for an extensible part 16 included in the extensible part 15. Specifically, as the constituent material of the sheet piece for an extensible part 16, for example, an extensible film can be used. Examples of the film include a single-layer film and a multi-layer film such as a coextruded film. The material constituting the extensible film is preferably a polyolefin such as a linear low-density polyethylene. At least one material selected from the group consisting of polyvinyl chloride, ethylene-vinyl acetate copolymers, and polyvinyl alcohol is also preferred. Use of a material having a permanent deformation of at least 50%, more preferably at least 70%, is also advantageous.

In order to linearly spread the disposal tape 10 folded in three and to successfully extend the extensible part 15, not only easy extension of the extensible part 15 but also the peel strength between the extensible part 15 and the fixing part 12 in an unextended state and the peel strength between the attachment part 18 and the extensible part 15 in an unextended state are important. From this viewpoint, the peel strength between the extensible part 15 and the fixing part 12 is preferably lower than the tensile strength at which the extensible part 15 starts to extend. Accordingly, before the extensible part 15 starts to extend when the disposal tape 10 is pulled in the longitudinal direction X1, the extensible part 15 is released from the fixing part 12, and the disposal tape 10 is easily spread. From this viewpoint, the peel strength between the extensible part 15 and the fixing part 12 is preferably not more than 95% of the tensile strength at which the extensible part 15 starts to extend, more preferably not more than 90%, and even more preferably not more than 85%. From the viewpoint of preventing unintentional release when extension is not needed, the peel strength is preferably 40% or more.

The peel strength between the extensible part 15 and the fixing part 12 is preferably 5.5 N or less, more preferably 5.0 N or less, and even more preferably 4.5 N or less on the condition in which the peel strength is lower than the tensile strength at which the extensible part 15 starts to extend. The lower limit of the peel strength is preferably 0.3 N or more, more preferably 0.5 N or more, and even more preferably 1.0 N or more from the viewpoint of maintaining the disposal tape 10 folded in three. The peel strength between an extensible part 15 and a fixing part 12 is determined independent of the width of a disposal tape 10.

From the above viewpoint, as the adhesive part for an extensible part 17 for releasably joining the extensible part 15 to the fixing part 12, for example, a rubber-based pressure-sensitive adhesive or an acrylic pressure-sensitive adhesive is typically used, and a rubber-based pressure-sensitive adhesive is preferably used. Examples of the rubber-based pressure-sensitive adhesive include synthetic rubbers such as a styrene-butadiene block copolymer and a hydrogenated styrene-butadiene block copolymer and a blend of such a synthetic rubber with a resin. Heat sealing, a hot-melt pressure-sensitive adhesive, or a pressure-sensitive adhesive including a melt-blown or fibrous pressure-sensitive adhesive or adhesive is also preferably used.

The adhesive part for an extensible part 17 included in the extensible part 15 preferably has an adhesive power that decreases as the extensible part 15 extends. Accordingly, a hand or dust is effectively prevented from unintentionally adhering to the adhesive part for an extensible part 17 in the extensible part 15 in an extended state, and this helps disposal operation of the diaper 1. From this viewpoint, for the adhesive part for an extensible part 17, the ratio of the adhesive power P2 of the extensible part 15 in a four-time extended state to the adhesive power P1 in an unextended state, P2/P1, is preferably 1.0 or less, more preferably 0.8 or less, and even more preferably 0.5 or less. From the same viewpoint, the ratio is preferably 0 or more. The adhesive power of an adhesive part for an extensible part 17 is determined independent of the width of a disposal tape 10.

The adhesive power of an adhesive part for an extensible part 17 is determined by the following method. A disposal tape 10 in an unextended state is linearly spread, and the disposal tape 10 is fixed to an acrylic plate such that the adhesive part for an extensible part 17 faces vertically upward. In the condition, a polyethylene film is stacked on the adhesive part for an extensible part 17 and is bonded by one reciprocation of a roller having a width of 25 mm, a diameter of 90 mm, and a mass of 1,200 g. Next, the film is pulled in a perpendicular direction to the adhesive part for an extensible part 17, and the film is released. The maximum power before release is defined as the adhesive power of the adhesive part for an extensible part 17. The peeling rate is set at 300 mm/min, and a tensile tester is used for the measurement. Substantially the same measurement is also performed for a disposal tape 10 in an extended state.

In order to reduce the adhesive power of the adhesive part for an extensible part 17 as the extensible part 15 extends, for example, the adhesive part for an extensible part 17 is preferably intermittently provided along the longitudinal direction X1 of the extensible part 15. This structure can reduce the area of the adhesive part for an extensible part 17, more effectively prevents a hand or dust from unintentionally adhering, and further helps disposal operation of the diaper 1. From this viewpoint, in an unextended state, the proportion of the area of the adhesive part for an extensible part 17 in the second face 16b that is included in the sheet piece for an extensible part 16 and faces the fixing part is preferably 70% or less, more preferably 60% or less, and even more preferably 50% or less. The proportion of the area of the adhesive part for an extensible part 17 is preferably 10% or more, more preferably 20% or more, and even more preferably 30% or more. In particular, the proportion of the area of the adhesive part for an extensible part 17 is preferably 10% or more and 70% or less, more preferably 20% or more and 60% or less, and even more preferably 30% or more and 50% or less. In this case, the area of the second face 16b excludes the region to which the folded part 13' of the sheet piece for a fixing part 13 is joined.

When the adhesive part for an extensible part 17 is intermittently provided on the second face 16b of the sheet piece for an extensible part 16 in the extensible part 15, and the extensible part 15 is extended, the side without the adhesive part for an extensible part 17 more easily extends than the side with the adhesive part for an extensible part 17 due to the presence of the adhesive part for an extensible part 17. This structure can further reduce the area of the adhesive part for an extensible part 17 in an extended state of the extensible part 15, even more effectively prevents a hand or dust from unintentionally adhering, and still further helps disposal operation of the diaper 1.

In place of the intermittent arrangement of the adhesive part for an extensible part 17 in the longitudinal direction X1 of the extensible part 15, the following method can also reduce the adhesive power of the adhesive part for an extensible part 17 as the extensible part 15 extends. For example, the adhesive part for an extensible part 17 may have a lower deformation amount than the deformation amount of the sheet piece for an extensible part 16 when the extensible part 15 is extended. Accordingly, the adhesive part for an extensible part 17 is likely to be partially broken before breakage of the sheet piece for an extensible part 16 when the extensible part 15 is extended. By the partial breakage of the adhesive part for an extensible part 17, regions without the adhesive part for an extensible part 17 are formed on the second face 16b of the sheet piece for an extensible part 16, and the area of the adhesive part for an extensible part 17 can be reduced in an extended state of the extensible part 15. Hence, the adhesive power of the adhesive part for an extensible part 17 can be reduced as the extensible part 15 extends.

Alternatively, on the sheet piece for an extensible part 16 or the adhesive part for an extensible part 17, or between the sheet piece for an extensible part 16 and the adhesive part for an extensible part 17, microcapsules containing an adhesive power lowering agent may be provided. The adhesive power lowering agent can be discharged from the microcapsules by deformation strain or the like of the sheet piece for an extensible part 16, thus reducing the adhesive power of the adhesive part for an extensible part 17 as the extensible part 15 extends. As the adhesive power lowering agent, a hydrocarbon or a mixture of a hydrocarbon and a surfactant can be used. As the microcapsules, microcapsules including a synthetic polymer such as polyvinylpyrrolidone, carboxymethyl cellulose, and gelatin or microcapsules including a synthetic resin such as a melamine/formaldehyde resin and an isocyanate resin can be used, for example.

The peel strength between an extensible part 15 and a fixing part 12 is determined by the following method. First, the fixing part 12 of a disposal tape 10 folded in three is fixed to an acrylic plate. Next, the attachment part 18 is released from the extensible part 15, and an assistant paper is bonded to the adhesive part for an attachment part 20 of the attachment part 18. The assistant paper has the same width as the width of the sheet piece for an attachment part 19. The acrylic plate to which the disposal tape 10 is fixed is attached to a chuck of a tensile tester, and the assistant paper is attached to a chuck at the opposite side. The chucks are moved at a rate of 300 mm/min in the spreading direction of the disposal tape 10 (180° direction) to release the extensible part 15 from the fixing part 12. The maximum force observed during the measurement is defined as the peel strength between the extensible part 15 and the fixing part 12.

The peel strength between the attachment part 18 and the extensible part 15 is preferably lower than the tensile strength at which the extensible part 15 starts to extend. Accordingly, when the disposal tape 10 is pulled in the longitudinal direction X1, the attachment part 18 is released from the extensible part 15, and the disposal tape 10 is likely to be spread before the extensible part 15 starts to extend. From this viewpoint, the peel strength between the attachment part 18 and the extensible part 15 is preferably not more than 80% of the tensile strength at which the extensible part 15 starts to extend, more preferably not more than 70%, and even more preferably not more than 60%. From the viewpoint of preventing unintentional release, the peel strength is preferably not less than 40%. The peel strength between an attachment part 18 and an extensible part 15 is determined independent of the width of a disposal tape 10.

The peel strength between the attachment part 18 and the extensible part 15 is preferably 4.0 N or less, more preferably 3.0 N or less, and even more preferably 2.0 N or less on the condition in which the peel strength is lower than the tensile strength at which the extensible part 15 starts to extend. When the upper limit of the peel strength is the above value, the disposal tape 10 is easily spread, and the attachment part 18 can have an enough attachment force. The lower limit of the peel strength is preferably 0.3 N or more, more preferably 0.5 N or more, and even more preferably 1.0 N or more from the viewpoint of maintaining the disposal tape 10 folded in three.

From the above viewpoint, as the adhesive part for an attachment part 20 for releasably joining the attachment part 18 to the extensible part 15, for example, a rubber-based pressure-sensitive adhesive or an acrylic pressure-sensitive adhesive is typically used, and a rubber-based pressure-sensitive adhesive is preferably used. Examples of the rubber-based pressure-sensitive adhesive include synthetic rubbers such as a styrene-butadiene block copolymer and a hydrogenated styrene-butadiene block copolymer and a blend of such a synthetic rubber with a resin.

The peel strength between an attachment part 18 and an extensible part 15 is determined by the following method. First, the fixing part 12 of a disposal tape 10 folded in three is fixed to an acrylic plate. Next, the tab 21 of the attachment part 18 is attached to a chuck. When the tab is difficult to hold, an assistant paper or the like may be bonded to the tab 21 and may be attached to a chuck. In this case, the assistant paper has the same width as the width of the sheet piece for an attachment part 19. The acrylic plate to which the disposal tape 10 is fixed is attached to a chuck of a tensile tester, and the tab 21 (the assistant paper) is attached to a chuck at the opposite side. The chucks are moved at a rate of 300 mm/min in the spreading direction of the disposal tape 10 (180° direction) to release the attachment part 18 from the extensible part 15. The maximum force observed during the measurement is defined as the peel strength between the attachment part 18 and the extensible part 15. As the assistant paper, an unstretchable film, paper, or the like having strength is used.

The diaper 1 of the embodiment may include one or a plurality of elastic members extending in the width direction Y For example, an elastic member extending in the width direction Y may be provided in an extended state near the waist opening 1W or in a below-waist region between the waist opening 1W and the leg openings 1L. By providing such an elastic member, the diaper 1 can more closely fit the body of a wearer, thus the diaper 1 gives good wearing comfort, and excrements can be effectively prevented from leaking. However, provided elastic members may form unevenness on the outer surface of the diaper 1 in a region in which the elastic members are provided. When the disposal tape 10 is intended to be fixed in the region in which such unevenness is formed, a clearance may be formed between the fixing part 12 and the outer surface of the diaper 1 to interfere with reliable fixation, and the disposal tape 10 may be unintentionally released unfortunately. In order to prevent such a disadvantage, the disposal tape 10 is advantageously provided on the outer surface of the diaper 1 at a position over which no elastic member crosses in a planar view.

In the diaper 1 of the present embodiment, waist gathers 3 and leg gathers 4 can be formed in marginal regions of the diaper 1. In the description, the marginal region is a region at the waist edge side of the absorbent member 2. These gathers are formed in a low rigidity region in many cases from the viewpoint of achieving satisfactory elasticity. In the description, the low rigidity region has a lower rigidity than that of a comparatively high rigidity region where the absorbent member 2 overlaps. When the disposal tape 10 is provided in such a low rigidity region, the disposal tape 10 may be difficult to spread at the time of disposal of the diaper 1 due to a low rigidity of the low rigidity region. In order to prevent such a disadvantage, the disposal tape 10 is preferably provided in a high rigidity region located inside the low rigidity region. Examples of the high rigidity region include a region overlapping with the absorbent member 2 in the diaper 1 viewed in the thickness direction. For example, in the diaper 1 shown in Fig. 1, the disposal tape 10 is preferably provided at a position lower than the lower end of the waist gathers 3, or a position lower than the upper end of the absorbent member 2, and at a position overlapping with the absorbent member 2.

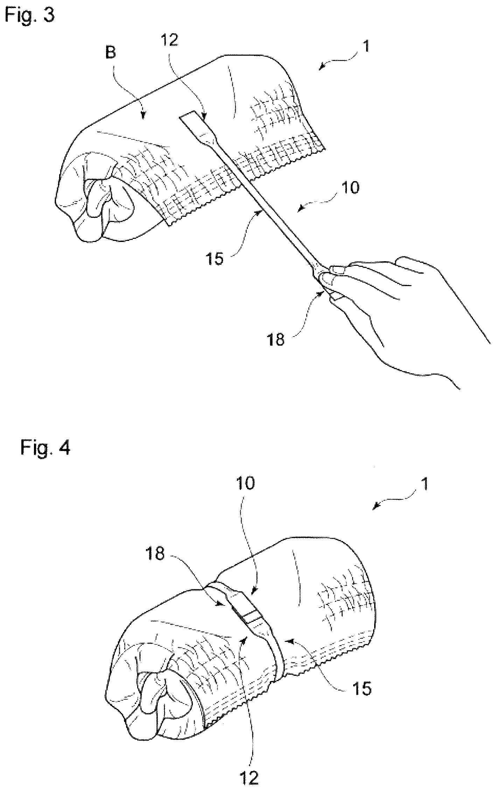

Fig. 3 shows when the diaper 1 of the embodiment is made in a disposal shape. As shown in the figure, when the diaper 1 is disposed, the diaper 1 is rolled up from the crotch portion C toward the waist opening 1W so that the front portion A of the diaper 1 faces inward, forming a rolled shape. In the rolling completion state, the disposal tape 10 provided on the rear portion B is exposed. In the state, the tab 21 of the disposal tape 10 is held and pulled by fingers, and while the disposal tape 10 folded in three is released and linearly spread, the extensible part 15 is extended. As described above, the extensible part 15 easily extends to increase the length. Next, as shown in Fig. 4, the rolled diaper is fastened with the extended disposal tape 10, and the adhesive part for an attachment part 20 of the attachment part 18 located in the leading end region of the disposal tape 10 is attached to the outer surface of the diaper 1 to maintain the rolled shape, or the disposal shape.

The present invention has been described on the basis of the preferred embodiments thereof, but the present invention is not limited to the above embodiments. For example, in the above embodiment, the disposal tape 10 is provided in the rear portion B of the diaper 1, but the position where the disposal tape 10 is provided is not limited to this and may be, for example, the front portion A or the crotch portion C. In the above embodiment, the disposal tape 10 is provided at substantially the center of the diaper 1 in the width direction Y, but the disposal tape 10 may be provided at any position and may be provided in a right or left region of the diaper 1, for example. In the above embodiment, the disposal tape 10 is provided so that the longitudinal direction X1 thereof is coincident with the longitudinal direction X of the diaper 1, but the direction of the disposal tape 10 is not limited to this. For example, the disposal tape 10 may be provided so that the longitudinal direction X1 thereof is coincident with the width direction Y of the diaper 1.

The pull-on garment of the present invention is not limited to the pull-on disposable diaper having the shape shown in Fig. 1 and may have another shape as long as the garment is put on the below waist of a wearer.

### Example

The present invention will next be described in further detail with reference to examples. However, the scope of the present invention is not limited to the examples.

### [Example 1]

As a pull-on disposable diaper, a pull-on disposable diaper manufactured by Kao Corporation, Merries Pants (manufactured in 2017, registered trademark), was used. From the diaper, the disposal tape was removed, and a disposal tape having specifications shown in Table 1 and having the structure shown in Fig. 2 was attached, giving a pull-on disposable diaper having the shape shown in Fig. 1.

### [Examples 2 and 3 and Comparative Examples 1 and 2]

In Example 1, disposal tapes having specifications shown in Table 1 were used. Pull-on disposable diapers were prepared in the same manner as in Example 1 except the disposal tapes.

### [Evaluation]

For the pull-on disposable diapers prepared in Examples and Comparative Examples, easiness of diaper disposal by a disposal tape was evaluated by the following method. The results are shown in Table 1.

### [Easiness of diaper disposal]

For the diapers prepared in Examples and Comparative Examples, the operation of rolling a diaper and fixing the diaper with a disposal tape was performed by 20 monitors. Easiness of the operation was evaluated and was classified on the basis of the following criteria.

### <Evaluation criteria>

A: Not less than 80% of the monitors reported easy disposal due to an extended disposal tape.
B: Not less than 50% and less than 80% of the monitors reported easy disposal due to an extended disposal tape.
C: Less than 50% of the monitors reported easy disposal due to an extended disposal tape.

**Table 1**

| | | Example 1 | Example 2 | Example 3 | Comparative Example 1 | Comparative Example 2 |
|---|---|---|---|---|---|---|
| Fixing part 12 | Width (mm) | 15 | 15 | 15 | 15 | 15 |
| | Length (mm) | 45 | 45 | 45 | 51 | 55 |
| | Material of sheet piece 13 | Polyolefin | Polyolefin | Polyolefin | Polyolefin | Polyolefin |
| | Material of adhesive part 14 | Synthetic rubber (SIS) | Synthetic rubber (SIS) | Synthetic rubber (SIS) | Styrenic block copolymer | Synthetic rubber (SIS) |
| | | | | | Hydrogenated terpene resin | |
| | | | | | Terpene resin | |
| Extensible part 15 | Width (mm) | 15 | 15 | 15 | 15 | 15 |
| | Length (mm) | 43 | 43 | 43 | 50 | 50 |
| | Material of sheet piece 16 | Polyurethane film | Polyurethane film | Polyurethane film | Polyolefin | Polyolefin |
| | Material of adhesive part 17 | Synthetic rubber (SIS) | Synthetic rubber (SIS) | Synthetic rubber (SIS) | Polypropylene | Synthetic rubber (SIS) |
| | Tensile strength at which extension starts (N) | 6.1 | 5.7 | 6.3 | 15.1 | 15.4 |
| | Extended length without breakage (mm) | 160 | 130 | 100 | 300 | 100 |
| | Length after extension L2 /length before extension L1 | 5 | 4 | 3 | 10 | 3 |
| | Intermittent adhesive part along longitudinal direction | with | with | with | with | without |
| | Proportion of area of adhesive part (%) | 50 | 70 | 70 | 50 | 100 |
| | Adhesive power in unextended state P1 (N) | 2.2 | 2.4 | 2.5 | 0 | 3.1 |
| | Adhesive power in extended state P2 (N) | 0.8 | 1.3 | 1.4 | 0 | 3.1 |
| | P2/P1 | 0.4 | 0.5 | 0.6 | - | 1.0 |
| Attachment part 18 | Width (mm) | 15 | 15 | 15 | 15 | 15 |
| | Length (mm) | 32 | 32 | 32 | 55 | 42 |
| | Tensile strength at which extension starts (N) | 15.2 | 15.3 | 15.0 | 15.1 | 15.2 |
| | Material of sheet piece 19 | Polyolefin | Polyolefin | Polyolefin | Polyolefin | Polyolefin |
| | Material of adhesive part 20 | Synthetic rubber (SIS) | Synthetic rubber (SIS) | Synthetic rubber (SIS) | Styrenic block copolymer | Synthetic rubber (SIS) |
| | | | | | Hydrogenated terpene resin | |
| | | | | | Terpene resin | |
| Peel strength between extensible part 15 and fixing part 12 (N) | | 1.2 | 1.3 | 1.1 | 3.5 | 1.9 |
| Peel strength between attachment part 18 and extensible part 15 (N) | | 3.5 | 4.0 | 3.2 | 0.6 | 5.0 |
| Easiness of disposal | | A | B | B | C | C |

As apparent from the results shown in Table 1, the diapers in Examples have excellent operability when the diapers are rolled and disposed as compared with the diapers in Comparative Examples.

### Industrial Applicability

As specifically described above, according to the present invention, an extended disposal tape enables an improvement in easiness of disposal when a pull-on disposable diaper is disposed.

## Claims

1. A pull-on disposable diaper (1) comprising:
a waist opening (1W);
a pair of leg openings (1L);
a front portion (A) to be placed on a front of a wearer wearing the diaper (1);
a rear portion (B) to be placed on a rear of the wearer; and
a disposal tape (10) for disposing the diaper arranged on an outer surface of the disposable diaper (1) to maintain a disposal shape of the disposable diaper (1), wherein
the disposal tape (10) includes a fixing part (12) fixed to the outer surface of the disposable diaper (1), an extensible part (15), and an attachment part (18) in sequence in a longitudinal direction of the disposal tape (10) and is folded in three stacked in the sequence,
in the disposal tape (10) folded in three, the attachment part (18) and the extensible part (15) are releasably joined, and the extensible part (15) and the fixing part (12) are also releasably joined,
the attachment part (18) starts to extend at a tensile strength higher than a tensile strength at which the extensible part (15) starts to extend,
a peel strength between the extensible part (15) and the fixing part (12) is lower than the tensile strength at which the extensible part (15) starts to extend,
a peel strength between the attachment part (18) and the extensible part (15) is lower than the tensile strength at which the extensible part (15) starts to extend,
the tensile strength at which the extensible part (15) starts to extend is 6.5 N or less, and
the peel strength between the attachment part (18) and the extensible part (15) is 4.0 N or less,
wherein the peel strength between the extensible part (15) and the fixing part (12) is measured with the method as described in paragraph [0041] of the A1-publication, the peel strength between the attachment part (18) and the extensible part (15) is measured with the method as described in paragraph [0045] of the A1-publication, and the tensile strength is measured with the method as described in paragraph [0027] of the A1-publication.

2. The pull-on disposable diaper according to claim 1, wherein the disposable diaper (1) includes an elastic member extending in a width direction, and
the disposal tape (10) is provided on the outer surface of the disposable diaper (1) at a position over which the elastic member does not cross.

3. The pull-on disposable diaper according to claim 1 or 2, wherein the disposal tape (10) is provided in a high rigidity region located inside a low rigidity region that is located in a marginal region of the disposable diaper (1).

4. The pull-on disposable diaper according to any one of claims 1 to 3, wherein the extensible part (15) has such an extensibility that an extended length without breakage is 150 mm or more.

5. The pull-on disposable diaper according to any one of claims 1 to 4, wherein the extensible part (15) has such an extensibility that a ratio of a length after extension L2 to a length before extension L1, L2/L1, is 4 or more.

6. The pull-on disposable diaper according to any one of claims 1 to 5, wherein the extensible part (15) has an adhesive part on a face facing the fixing part (12), and
an adhesive power by the adhesive part reduces as the extensible part extends.

7. The pull-on disposable diaper according to claim 6, wherein the adhesive part is intermittently provided along a longitudinal direction of the extensible part (16).

8. The pull-on disposable diaper according to claim 7, wherein the extensible part (15) has a higher extensibility at a side without the adhesive part than a side with the adhesive part.

9. The pull-on disposable diaper according to any one of claims 1 to 8, wherein the extensible part (15) includes a material having a permanent deformation of at least 50%.

10. The pull-on disposable diaper according to any one of claims 1 to 9, wherein the extensible part (15) starts to extend at a tensile strength of 1.0 N or more.

11. The pull-on disposable diaper according to any one of claims 1 to 10, wherein the peel strength between the extensible part (15) and the fixing part (12) is not more than 95% of the tensile strength at which the extensible part (15) starts to extend.

12. The pull-on disposable diaper according to any one of claims 1 to 11, wherein the peel strength between the extensible part (15) and the fixing part (12) is 5.5 N or less on the condition in which the peel strength is lower than the tensile strength at which the extensible part (15) starts to extend.

13. The pull-on disposable diaper according to any one of claims 1 to 12, wherein the extensible part (15) includes a sheet piece for an extensible part (16),
the extensible part (15) includes an adhesive part for an extensible part (17),
the sheet piece for an extensible part (16) has a first face (16a) and a second face (16b), and
in an unextended state, a proportion of an area of the adhesive part for an extensible part (17) in the second face (16b) that is on the sheet piece for an extensible part (16) and faces the fixing part is 70% or less.

14. The pull-on disposable diaper according to any one of claims 1 to 13, wherein the peel strength between the attachment part (18) and the extensible part (15) is not more than 80% of the tensile strength at which the extensible part (15) starts to extend.

15. The pull-on disposable diaper according to any one of claims 1 to 14, wherein the peel strength between the attachment part (18) and the extensible part (15) is 3.0 N or less.

## Patentansprüche

1. Wegwerfschlupfwindel (1), die aufweist:
eine Taillenöffnung (1W);
ein Paar von Beinöffnungen (1L);
einen vorderen Abschnitt (A), der an der Vorderseite eines Trägers angeordnet werden soll, der die Windel (1) trägt;
einen hinteren Abschnitt (B), der an der Rückseite des Trägers angeordnet werden soll; und
ein Entsorgungsband (10) zum Entsorgen der Windel, das an einer Außenfläche der Wegwerfwindel (1) angeordnet ist, um eine Entsorgungsform der Wegwerfwindel (1) zu erhalten, wobei
das Entsorgungsband (10) einen Fixierungsteil (12), der an der Außenfläche der Wegwerfwindel (1) befestigt ist, einen dehnbaren Teil (15) und einen Befestigungsteil (18) in einer Längsrichtung des Entsorgungsbandes (10) hintereinander aufweist und in der Reihenfolge dreifach gestapelt gefaltet ist,
in dem dreifach gefalteten Entsorgungsband (10) der Befestigungsteil (18) und der dehnbare Teil (15) lösbar verbunden sind, und der dehnbare Teil (15) und der Fixierungsteil (12) ebenfalls lösbar verbunden sind,
der Befestigungsteil (18) sich bei einer Zugfestigkeit zu dehnen beginnt, die höher ist als eine Zugfestigkeit, bei der sich der dehnbare Teil (15) zu dehnen beginnt,
eine Schälfestigkeit zwischen dem dehnbaren Teil (15) und dem Fixierungsteil (12) geringer ist als die Zugfestigkeit, bei der sich der dehnbare Teil (15) zu dehnen beginnt, eine Schälfestigkeit zwischen dem Befestigungsteil (18) und dem dehnbaren Teil (15) geringer ist als die Zugfestigkeit, bei der sich der dehnbare Teil (15) zu dehnen beginnt, die Zugfestigkeit, bei der sich der dehnbare Teil (15) zu dehnen beginnt, 6,5 N oder weniger beträgt, und
die Schälfestigkeit zwischen dem Befestigungsteil (18) und dem dehnbaren Teil (15) 4,0 N oder weniger beträgt,
wobei die Schälfestigkeit zwischen dem dehnbaren Teil (15) und dem Fixierungsteil (12) mit dem in Absatz [0041] der A1-Veröffentlichung beschriebenen Verfahren gemessen wird, die Schälfestigkeit zwischen dem Befestigungsteil (18) und dem dehnbaren Teil (15) mit dem in Absatz [0045] der A1-Veröffentlichung beschriebenen Verfahren gemessen wird, und die Zugfestigkeit mit dem in Absatz [0027] der A1-Veröffentlichung beschriebenen Verfahren gemessen wird.

2. Wegwerfschlupfwindel nach Anspruch 1, wobei die Wegwerfwindel (1) ein elastisches Element aufweist, das sich in einer Breitenrichtung erstreckt, und
das Entsorgungsband (10) auf der Außenfläche der Wegwerfwindel (1) an einer Position vorgesehen ist, die das elastische Element nicht kreuzt.

3. Wegwerfschlupfwindel nach Anspruch 1 oder 2, wobei das Entsorgungsband (10) in einem Bereich mit hoher Steifigkeit vorgesehen ist, der sich innerhalb eines Bereichs mit geringer Steifigkeit befindet, der sich in einem Randbereich der Wegwerfwindel (1) befindet.

4. Wegwerfschlupfwindel nach einem der Ansprüche 1 bis 3, wobei der dehnbare Teil (15) eine solche Dehnbarkeit aufweist, dass eine ausgezogene Länge ohne Bruch 150 mm oder mehr beträgt.

5. Wegwerfschlupfwindel nach einem der Ansprüche 1 bis 4, wobei der dehnbare Teil (15) eine solche Dehnbarkeit aufweist, dass das Verhältnis der Länge nach der Dehnung L2 zu der Länge vor der Dehnung L1, L2/L1, 4 oder mehr beträgt.

6. Wegwerfschlupfwindel nach einem der Ansprüche 1 bis 5, wobei der dehnbare Teil (15) einen Klebeteil auf einer dem Fixierungsteil (12) zugewandten Seite aufweist, und
die Klebekraft des Klebeteils mit zunehmender Ausdehnung des dehnbaren Teils abnimmt.

7. Wegwerfschlupfwindel nach Anspruch 6, wobei der klebende Teil intermittierend entlang einer Längsrichtung des dehnbaren Teils (16) vorgesehen ist.

8. Wegwerfschlupfwindel nach Anspruch 7, wobei der dehnbare Teil (15) an einer Seite ohne den Klebeteil eine höhere Dehnbarkeit aufweist als an einer Seite mit dem Klebeteil.

9. Wegwerfschlupfwindel nach einem der Ansprüche 1 bis 8, wobei der dehnbare Teil (15) ein Material mit einer bleibenden Verformung von mindestens 50% aufweist.

10. Wegwerfschlupfwindel nach einem der Ansprüche 1 bis 9, wobei sich der dehnbare Teil (15) bei einer Zugfestigkeit von 1,0 N oder mehr zu dehnen beginnt.

11. Wegwerfschlupfwindel nach einem der Ansprüche 1 bis 10, wobei die Schälfestigkeit zwischen dem dehnbaren Teil (15) und dem Fixierungsteil (12) nicht mehr als 95% der Zugfestigkeit beträgt, bei der sich der dehnbare Teil (15) zu dehnen beginnt.

12. Wegwerfschlupfwindel nach einem der Ansprüche 1 bis 11, wobei die Schälfestigkeit zwischen dem dehnbaren Teil (15) und dem Fixierungsteil (12) 5,5 N oder weniger beträgt, wenn die Schälfestigkeit geringer ist als die Zugfestigkeit, bei der sich der dehnbare Teil (15) zu dehnen beginnt.

13. Wegwerfschlupfwindel nach einem der Ansprüche 1 bis 12, wobei der dehnbare Teil (15) ein Bahnstück für einen dehnbaren Teil (16) aufweist,
der dehnbare Teil (15) einen Klebeteil für einen dehnbaren Teil (17) enthält,
das Bahnstück für einen dehnbaren Teil (16) eine erste Fläche (16a) und eine zweite Fläche (16b) aufweist, und
in einem ungedehnten Zustand ein Anteil einer Fläche des Klebeteils für einen dehnbaren Teil (17) in der zweiten Fläche (16b), die sich auf dem Bahnstück für einen dehnbaren Teil (16) befindet und dem Fixierungsteil zugewandt ist, 70% oder weniger beträgt.

14. Wegwerfschlupfwindel nach einem der Ansprüche 1 bis 13, wobei die Schälfestigkeit zwischen dem Befestigungsteil (18) und dem dehnbaren Teil (15) nicht mehr als 80% der Zugfestigkeit beträgt, bei der sich der dehnbare Teil (15) zu dehnen beginnt.

15. Wegwerfschlupfwindel nach einem der Ansprüche 1 bis 14, wobei die Schälfestigkeit zwischen dem Befestigungsteil (18) und dem dehnbaren Teil (15) 3,0 N oder weniger beträgt.

## Revendications

1. Couche-culotte jetable (1), comprenant :
une ouverture de taille (1W) ;
une paire d'ouvertures de jambe (1L) ;
une partie avant (A) à mettre en place sur le devant du porteur de la couche (1) ;
une partie arrière (B) à mettre en place dans le dos du porteur ; et
une bande jetable (10) pour se débarrasser de la couche, disposée sur la surface extérieure de la couche jetable (1) pour obtenir une forme permettant de jeter la couche jetable (1), où
la bande jetable (10) comprend dans l'ordre une partie fixe (12) fixée à la surface extérieure de la couche jetable (1), une partie extensible (15), et une partie de fixation (18) dans le sens de la longueur de la bande jetable (10), et est pliée en trois couches dans cet ordre,
dans la bande jetable (10) pliée en trois, la partie de fixation (18) et la partie extensible (15) sont réunies de manière amovible, et la partie extensible (15) et la partie fixe (12) sont également réunies de manière amovible,
la partie de fixation (18) commence à s'étendre sous une force de traction supérieure à la force de traction sous laquelle la partie extensible (15) commence à s'étendre,
une résistance au décollement entre la partie extensible (15) et la partie fixe (12) est inférieure à la force de traction sous laquelle la partie extensible (15) commence à s'étendre,
une résistance au décollement entre la partie de fixation (18) et la partie extensible (15) est inférieure à la force de traction sous laquelle la partie extensible (15) commence à s'étendre,
la force de traction sous laquelle la partie extensible (15) commence à s'étendre est égale ou inférieure à 6,5 N, et
la résistance au décollement entre la partie de fixation (18) et la partie extensible (15) est égale ou inférieure à 4,0 N,
la résistance au décollement entre la partie extensible (15) et la partie fixe (12) étant mesurée au moyen du procédé décrit en paragraphe [0041] de la publication A1, la résistance au décollement entre la partie de fixation (18) et la partie extensible (15) étant mesurée au moyen du procédé décrit en paragraphe [0045] de la publication A1, et la force de traction étant mesurée au moyen du procédé décrit en paragraphe [0027] de la publication A1.

2. Couche-culotte jetable selon la revendication 1, où la couche jetable (1) comprend un élément élastique s'étendant dans le sens de la largeur, et
la bande jetable (10) est prévue sur la surface extérieure de la couche jetable (1) à un emplacement que ne croise pas l'élément élastique.

3. Couche-culotte jetable selon la revendication 1 ou la revendication 2, où la bande jetable (10) est prévue dans une zone à rigidité élevée située à l'intérieur d'une zone à faible rigidité située dans une zone marginale de la couche jetable (1).

4. Couche-culotte jetable selon l'une des revendications 1 à 3, où la partie extensible (15) a une extensibilité telle qu'une longueur d'extension sans rupture est égale ou supérieure à 150 mm.

5. Couche-culotte jetable selon l'une des revendications 1 à 4, où la partie extensible (15) a une extensibilité telle qu'un rapport L2/L1entre une longueur après extension L2 et une longueur avant extension L1 est égal ou supérieur à 4.

6. Couche-culotte jetable selon l'une des revendications 1 à 5, où la partie extensible (15) a une partie adhésive sur une face opposée à la partie fixe (12), et
un pouvoir d'adhérence de la partie adhésive diminue à mesure que la partie extensible s'étend.

7. Couche-culotte jetable selon la revendication 6, où la partie adhésive est prévue de manière discontinue dans le sens de la longueur de la partie extensible (16).

8. Couche-culotte jetable selon la revendication 7, où la partie extensible (15) a une extensibilité supérieure sur un côté sans la partie adhésive que sur un côté avec la partie adhésive.

9. Couche-culotte jetable selon l'une des revendications 1 à 8, où la partie extensible (15) comprend un matériau dont la déformation permanente est de 50 % au moins.

10. Couche-culotte jetable selon l'une des revendications 1 à 9, où la partie extensible (15) commence à s'étendre sous une force de traction égale ou supérieure à 1,0 N.

11. Couche-culotte jetable selon l'une des revendications 1 à 10, où la résistance au décollement entre la partie extensible (15) et la partie fixe (12) n'est pas supérieure à 95 % de la force de traction sous laquelle la partie extensible (15) commence à s'étendre.

12. Couche-culotte jetable selon l'une des revendications 1 à 11, où la résistance au décollement entre la partie extensible (15) et la partie fixe (12) est égale ou inférieure à 5,5 N à la condition que la résistance au décollement soit inférieure à la force de traction sous laquelle la partie extensible (15) commence à s'étendre.

13. Couche-culotte jetable selon l'une des revendications 1 à 12, où la partie extensible (15) comprend une pièce en feuille pour une partie extensible (16),
la partie extensible (15) comprend une partie adhésive pour une partie extensible (17), la pièce en feuille pour une partie extensible (16) a une première face (16a) et une deuxième face (16b), et
en état de non-extension, la proportion d'une surface de la partie adhésive pour une partie extensible (17) dans la deuxième face (16b) sur la pièce en feuille pour une partie extensible (16) et opposée à la partie fixe est égale ou inférieur à 70 %.

14. Couche-culotte jetable selon l'une des revendications 1 à 13, où la résistance au décollement entre la partie de fixation (18) et la partie extensible (15) n'est pas supérieure à 80 % de la force de traction sous laquelle la partie extensible (15) commence à s'étendre.

15. Couche-culotte jetable selon l'une des revendications 1 à 14, où la résistance au décollement entre la partie de fixation (18) et la partie extensible (15) est égale ou inférieure à 3,0 N.
